# FASCICULE DE BREVET EUROPEEN

(11) **EP 1 151 979 B1**
(45) Date de publication et mention de la délivrance du brevet: **15.02.2006**
(21) Numéro de dépôt: 01400977.3
(22) Date de dépôt: 17.04.2001
(51) Int. Cl.: C07B 37/02, C07C 2/66, C07C 6/12

(54) **Procedé de production conjointe ou non de monoalkylaromatiques, dialkyl aromatiques et trialkyl aromatiques**
Verfahren zur simultanen oder nicht simultanen Herstellung von monolalkylaromatischen, dialkylaromatischen und trialkylaromatischen Verbindungen
Process for the simultaneous or non-simultaneous production of monoalkylaromatic, dialkylaromatic and trialkylaromatic compounds

(30) Priorité: 02.05.2000 FR 0005677
(43) Date de publication de la demande: 07.11.2001
(73) Titulaire: Institut Français du Pétrole, 92852 Rueil-Malmaison Cedex (FR); Industrias Veneco C.A., 1060-A Caracas (VE)
(72) Inventeur: Briot, Patrick, 38260 Pommier de Beaurepaire (FR); Lew, Léon, Sabana Grande, Caracas 1050 (VE); Yout, Pierre, 38200 Vienne (FR); Hipeaux, Jean-Claude, 92700 Colombes (FR); Benazzi, Eric, 78400 Chatou (FR)

(56) Documents cités:
- US-A- 2 400 437

## Description

Le procédé objet de la présente demande est un procédé pour alkyler ou transalkyler des composés aromatiques en vue de produire des alkylaromatiques. Les monoalkyls aromatiques trouvent une utilisation dans la composition des essences ou des lessives, les dialkyls et trialkyls aromatiques dans le domaine des lubrifiants.

Ainsi, le procédé selon l'invention permet la production de mono, de di et de trialkyl aromatiques. Ainsi, ce procédé concerne l'alkylation de composés aromatiques (benzène, toluène, cumène..) par des agents alkylants choisis parmi les oléfines pour produire des monoalkyl aromatiques dont la chaîne aliphatique greffée comporte un nombre de carbone choisi de 2 à 20 atomes de carbone.

Ce procédé peut également produire aussi des dialkyls benzène c'est-à-dire des composés aromatiques où le noyau benzénique comporte deux chaînes paraffiniques dont le nombre d'atomes de carbone peut être identique ou différent. Chacune de ces chaînes aliphatiques peuvent contenir de 2 à 20 atomes de carbone. Dans le cas où il serait souhaité de produire des trialkyls aromatiques, il y a trois chaînes aliphatiques dont 2, par exemple, de longueurs identiques.

L'alkylation des composés aromatiques est connue depuis de nombreuses années.

Ainsi le brevet US-A-2,939,890 (Universal Oil Products Company), datant de 1960, revendique un procédé de synthèse du cumène en utilisant BF3 comme catalyseur.

Il est connu également le brevet US-A-3,173,965 (Esso Research), datant de 1965 qui revendique comme catalyseurs adéquats pour l'alkylation du benzène des acides du type AlCl3, AlBr3, FeCl3, SnCl4, BF3, H2SO4, P2O5 et H3PO4.

Dans le même ordre d'idée, les brevets US-A-4,148,834 (1979) et US-A-4,551,573 (1985) revendiquent l'utilisation pour le premier de HF lors de la première étape et AlCl3 ou AlBr3 dans la deuxième étape. Le second brevet, quant à lui, revendique, plus particulièrement, un mélange d'halogénures d'aluminium et d'iode élémentaire.

Le brevet US-A-3,251,897 revendique l'utilisation de zéolithes X et Y échangées aux terres rares pour la production de monoalkyl benzène (éthylbenzène, cumène..) et de diéthylbenzène.
Le brevet US-A-3,631,120 (1971) revendique l'utilisation de zéolithes principalement zéolithe Y ayant un rapport silice sur alumine de 4 à 4,9 pour la production de cumène.

Le brevet US-A-5,107,048 (1992) revendique l'utilisation de catalyseur amorphe du type silice promu acide de Lewis grace à BF3.

Le brevet US 2,400,437 décrit un procédé d'alkylation de composés aromatiques par des hydrocarbures oléfiniques au moyen de réacteurs disposés en série.

Le procédé, revendiqué par la demanderesse, concerne la production de monoalkyl benzène et/ou de dialkyl benzène et/ou de trialkyl benzène et possède comme originalités :
- un schéma de production qui est souple et qui permet de l'adapter aux besoins du marché ou aux possibilités d'approvisionnement,
- les catalyseurs utilisés ne sont ni dangereux ni toxiques comme le sont les halogénures d'aluminium ou HF,
- leur spécificité est telle qu'elle permet d'obtenir les produits de grande pureté,
- les catalyseurs, utilisés dans la présente invention, peuvent être régénérés par simple combustion à l'air ou sous oxygène, ou référencés par un traitement sou hydrogène.

L'invention se caractérise donc essentiellement par un procédé souple comportant normalement en série deux étapes combinées adaptées à la production, conjointe ou non, de mono, di et trialkylaromatiques, procédé dans lequel en fonction des produits désirés, il peut être mis en oeuvre soit les deux étapes soit la première soit la deuxième de ces deux étapes, chacune des étapes pouvant être mise hors circuit ou remise en marche à tout moment, en fonction des désidératats de la clientèle à approvisionner en mon, di ou trialkylaromatiques obtenu(s) par alkylation ou par transalkylation d'un hydrocarbure aromatique par un composé oléfinique.

Les trois types de produits susceptibles d'être produits, par le procédé dont la demanderesse revendique la propriété, correspondent aux formules chimiques génériques suivantes :

**Monoalkyl benzène :** 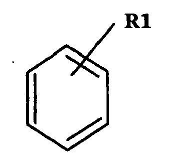
R1= chaîne aliphatique comportant alors de 2 à 20 atomes de carbone

**Dialkyl benzène :** 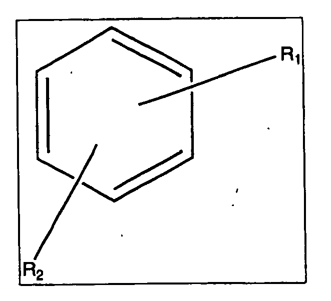
Ces chaînes alkyls possèdent chacune de 2 à 20 atomes de carbone. Ces chaînes peuvent être d'égales dimensions ou de longueur différentes.

Les produits obtenus sont constitués des trois isomères : ortho, méta et para.

**Trialkyls benzène :**

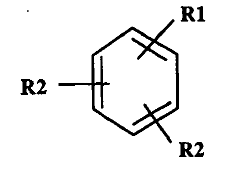

Ici les chaînes aliphatiques R1 et R2 peuvent être de longueur identique ou différentes et comprennent de 2 à 20 atomes de carbone.

### Description du procédé

Sur le procédé quatre types différents de production sont envisageables de façon séparée lorsqu'il n'est souhaité qu'un seul produit spécifique :
- cas 1 : production de diakyl benzène pour lesquels les chaînes aliphatiques sont de longueurs différentes,
- cas 2 : production de dialkyl benzène pour lesquels les chaînes aliphatiques sont de longueur identique,
- cas 3 : production de monoalkyl benzène,
- cas 4 : production de trialkyl benzène.

Deux types de réactions principales se produisent :

- alkylation :

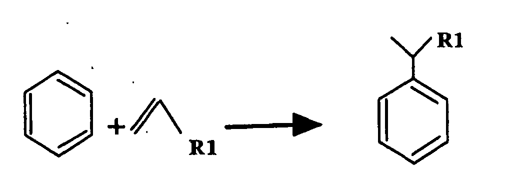

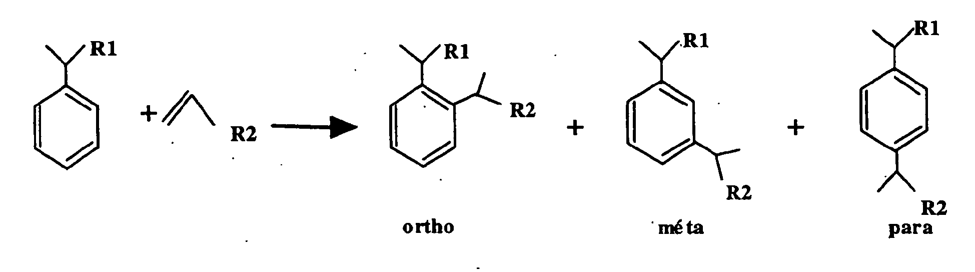

- trans-alkylation :

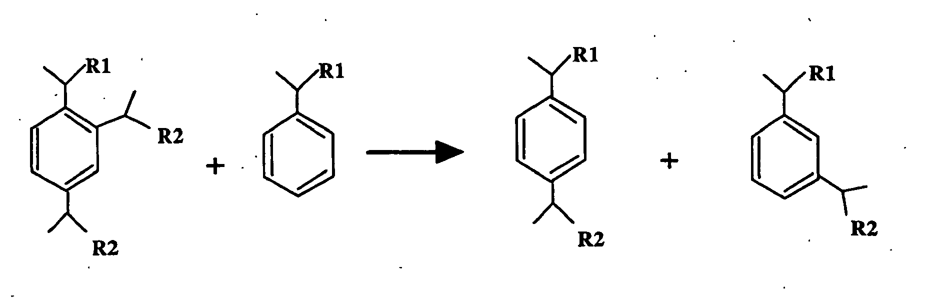

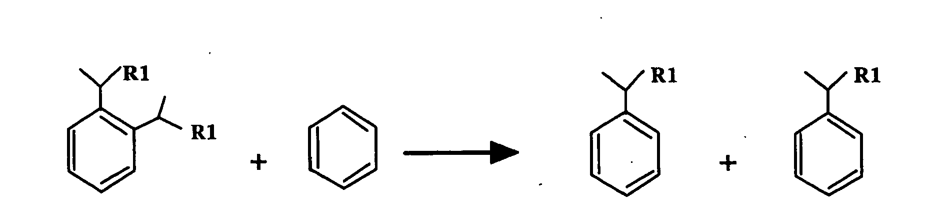

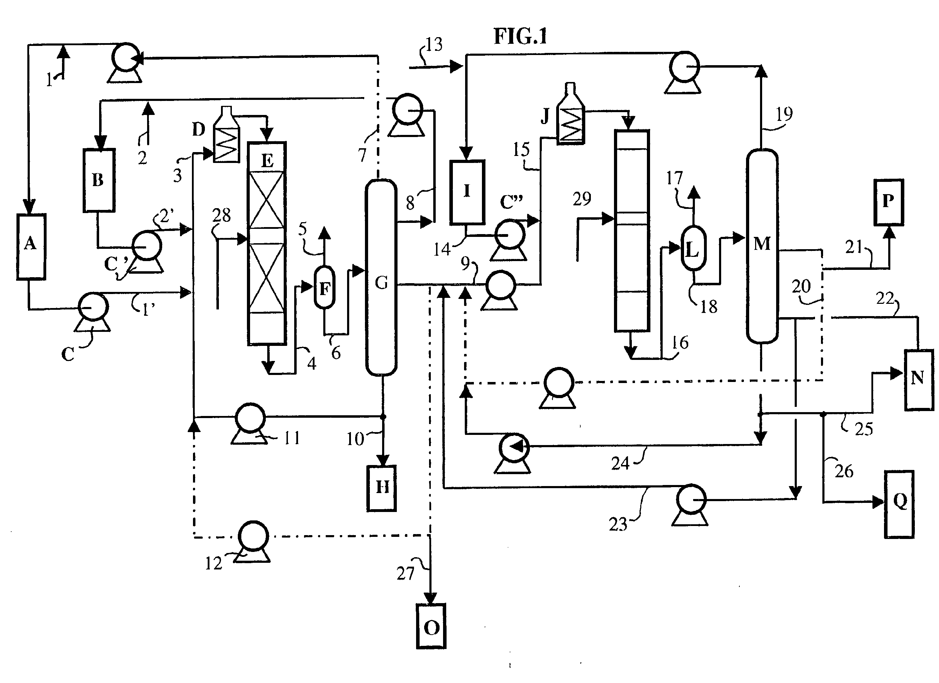

Un schéma simplifié du procédé est présenté sur la figure 1. Il est composé de deux étapes a et b qui peuvent être séparées ou imbriquées.

Dans la description du procédé qui est faite ci-après pour simplifier la compréhension, l'aromatique sera appelé benzène et l'agent alkylant sera appelé oléfine (oléfine 1 ou oléfine 2)

Dans l'étape a, l'aromatique (ou benzène) est transporté par la ligne 1 jusqu'à un bac d'alimentation A. L'agent alkylant (ou oléfine 1) est acheminé par la ligne 2 jusqu'à un bac d'alimentation B. Ces 2 produits sont ensuite envoyés dans une ligne 3 grace aux pompes C et C' et les lignes 1' et 2'. Les réactifs sont ensuite préchauffés à la température de réaction grâce à un four D. Les réactifs chauds sont ensuite injectés dans un réacteur E contenant le catalyseur n°1. Les caractéristiques de ce catalyseur seront détaillées ultérieurement. La réaction d'alkylation est exothermique d'où il peut être utile d'injecter une partie du composé aromatique (ou une partie de l'oléfine1) entre les différents lits du réacteur, s'il y a plusieurs lits, par la ligne 28. Les produits de la réaction sont acheminés par la ligne 4 à un séparateur F gaz-liquide permettant de séparer les gaz (ligne 5) et le liquide (ligne 6).

Cet effluent liquide est ensuite envoyé dans au moins une colonne de distillation (G) dans laquelle seront séparés :
- l'aromatique non converti (benzène) qui sera recyclé au bac A par la ligne 7,
- l'agent alkylant (oléfine 1) non converti pourra être recyclé au bac B par l'intermédiaire de la ligne 8,
- le monoalkyl aromatique (ou monoalkyl benzène) pourra être selon les cas :
   - cas 1 ou cas 4, défini plus haut: envoyé dans la seconde partie du procédé (c'est-à-dire dans la deuxième étape (b)) par la ligne 9,
   - cas 2 : recyclé à l'entrée du réacteur E par la ligne 12 pour subir une deuxième alkylation,
   - cas 3 : récupéré par la ligne 27 et stocké en tant que produit dans un bac O
- le dialkylaromatique (ou dialkylbenzène) pourra être selon les cas :
   - cas 1 ou 3 : recyclé par la ligne 11 puis 3 à l'entrée du réacteur afin de subir une trans-alkylation,
   - cas 2 : récupéré grâce à la ligne 10 et stocké dans un bac H.

Cette colonne de distillation G dans l'exemple pourra être avantageusement remplacée par une succession de colonne pouvant fonctionner à des pressions différentes (2 à 4 colonnes).

Dans l'étape b (cas 1), le monoalkyl aromatique (monoalkyl benzène) de la ligne 9 est mélangé avec l'agent alkylant 2 (ou oléfine 2) provenant de la ligne 13, du bac I par l'intermédiaire de la pompe C ". Le mélange est préchauffé dans le four J et pénètre à l'intérieur du réacteur K, contenant le second catalyseur (catalyseur n°2) par l'intermédiaire de la ligne 15. La réaction étant exothermique, il peut être judicieux d'injecter une partie de l'oléfine 2 entre les lits du réacteur (s'il y a plusieurs lits), par la ligne 29, pour contrôler les températures. Les effluents du réacteur (ligne 16) vont dans un séparateur gaz-liquide L. Les gaz sont évacués par la ligne 17. L'effluent liquide est envoyé par la ligne 18 à une colonne de distillation M. Les différents produits à l'issue de la distillation sont :
- en tête de colonne, l'agent alkylant 2 (ou oléfine 2) non converti qui sera recyclé (ligne 19) à entrée du réacteur par l'intermédiaire d'un bac d'alimentation I.
- le monoalkyl provenant du premier réacteur et non converti sera traité différement selon les cas. Dans le cas 1, il sera recyclé à l'entrée du deuxième réacteur à l'aide de la ligne 20. Dans le cas où il est souhaité une production conjointe de dialkylbenzène et de monoalkyl benzène, il peut être stocké dans un bac P par la ligne 21.
- Le dialkyl benzène dissymétrique est envoyé pour stockage dans un bac N par la ligne 22. Cependant, si la production souhaité est du trialkyls benzène (cas 4), il est possible de le recycler par la ligne 23 à l'entrée du deuxième réacteur pour subir une nouvelle alkylation.
- Le trialkyl benzène sera selon les cas : recyclé à l'entrée du deuxième réacteur par la ligne 24 pour subir une trans-alkylation, stocké comme produit dans un bac Q par la ligne 26, ou alors mélangé à la production de dialkyl dans le bac N par la ligne 25.

Comme précédemment, cette colonne de distillation M pourra être remplacée avantageusement par une succession de colonnes de distillation (2 à 4 colonnes) pouvant fonctionner à des pressions différentes.

Le catalyseur de l'étape a) peut être sous forme de billes, mais il est le plus souvent sous forme d'extrudés. Il est constitué d'un solide acide mélangé à une phase amorphe. Le solide acide en question est mis en forme grâce à une matrice, ce qui est une phase amorphe. Le solide acide est de préférence au moins une zéolithe. On choisira de préférence les zéolithes de type structural FAU et plus particulièrement la zéolithe Y, les zéolithes de type structural MOR (la zéolithe mordénite) synthétisés en milieu classique basique ou bien en milieu fuorure selon les procédés décrits dans les brevets EP-B-466 558 et EP-B-469 940, les zéolithes de type structural EUO, c'est-à-dire les zéolithes EU-1, ZSM-50, TPZ-3), la zéolithe NU-87 de type structural NES. Toutes ces zéolithes sont décrites dans le document «Atlas of zeolite Structure Types» W.M. Meier, D.H. Olso et Ch. Baerlocker, 1996 Elsevier. Parmi les zéolithes préférées et utilisables dans le catalyseur figurent aussi la zéolithe NU-86 décrite dans le brevet EP 463 768 A, la zéolithe NU-85 décrite dans le brevet EP 462 745 A, la zéolithe NU-88 décrite dans le brevet FR 2 752 567, et la zéolithe IM-5 décrite dans le brevet FR 2 754 809.
Ces zéolithes sont au moins en partie sous forme acide (H⁺), mais peuvent aussi contenir des cations autres que H⁺ et tels que les alcalins, alcalino-teneurs... La teneur en zéolithe dans le catalyseur est comprise entre 5 et 95 % poids de préférence entre 10 et 90 % par rapport au catalyseur final. Le rapport Si/Al global de ces zéolithes est compris entre 2,6 et 200 de préférence entre 5 et 100 et de manière encore plus préférée entre 5 et 80.
La matrice du catalyseur est un support choisi dans le groupe formé par l'alumine, la silice, la silice-alumine. L'alumine-oxyde de bore, la magnésie, la silice-magnésie, la zircone, l'oxyde de titane, l'argile, la silice-magnésie, la zircone, l'oxyde de titane, l'argile, ces composés étant utilisés seuls ou en mélanges. De manière préférée, on utilise un support d'alumine.

La surface B.E.T. du catalyseur 1 utilisé dans l'étape a) est comprise entre 50 et 900 m2/g de préférence entre 100 et 700 m²/g. Le rapport Na/Al du catalyseur final est inférieur à 5 % atomique et de préférence inférieur à 2 %.
Au catalyseur (zéolithe + matrice) peut éventuellement être ajouté des éléments métalliques comme par exemple, les métaux de la famille des terre rares, notamment le lanthane et le cérium, ou les métaux du groupe VIB de la classification périodique des éléments, comme le molybdène, ou des métaux nobles ou non nobles du groupe VIII, comme le platine, le palladium, le ruthénium, le rhodium, l'iridium, le fer et d'autres métaux comme le manganèse, le zinc, le magnésium. Ces éléments métalliques peuvent être déposés, soit sur la matrice, soit sur la phase zéolithique.
Un certain nombre de catalyseurs ont été testés. Le tableau 1 regroupe les caractéristiques de quelques uns.

**Tableau 1 : caractéristiques de quelques catalyseurs testés.**

| Référence catalyseurs | A | B | C | D |
|---|---|---|---|---|
| Nature zéolithe | Mordénite | Mordénite | Eu-1 | Nu-87 |
| Teneur pondérale en zéolithe (% poids) | 20% | 80% | 60% | 70%. |
| Teneur pondérale en alumine (% poids) | 80% | 20% | 40% | 30% |
| Si/Al (Fx) de la zéolithe | 38 | 40-50 | 36 | 33 |
| Surface B.E.T. (m2/g) | 475 | 483 | 444 | 483 |
| Na/Al (%atomique) | 0,8 | 1,02 | 0,1 | 0,6 |

Le catalyseur de l'étape b) peut être sous forme de billes, mais il est le plus souvent sous forme d'extrudés. Il est constitué d'un solide acide mélangé à une phase amorphe comme le catalyseur de l'étape a).

Le solide acide en question est mis en forme grâce à une matrice, qui est une phase amorphe. Le solide acide est de préférence au moins une zéolithe. On choisira de préférence les zéolithes de type structural FAU et plus particulièrement la zéolithe Y, les zéolithes de type structural MOR (zéolithe mordénite) synthétisées en milieu classique basique ou bien en milieu fluorure selon les procédés décrits dans les brevets EP-B-466 558 et EP-B-469 940, les zéolithes de type structural EU-O, c'est-à-dire les zéolithes EU-1, ZSM-50, TPZ-3), la zéolithe NU-87 de type structural NES.

Toutes ces zéolithes sont décrites dans le document « Atlas of Zeolite Structure Types » W.M. Meier, D.H. Olso et Ch. Baerlocker, 1996 Elsefier. Parmi les zéolithes préférées et utilisables dans le catalyseur figurent aussi la zéolithe NU-86 décrite dans le brevet EP 463 768 A, la zéolithe NU-85 décrite dans le brevet EP 462 745 A, la zéolithe NU-88 décrite dans le brevet FR 2 752 567, et la zéolithe IM-5 décrite dans le brevet le brevet FR 2 754 809.

Ces zéolithes sont au moins en partie sous forme acide (H⁺), mais peuvent aussi contenir des cations autres que H+ et tels que les alcalins, alcalino-terreux. La teneur en zéolithe dans le catalyseur est comprise entre 5 et 95% poids de préférence entre 10 et 90 % par rapport au catalyseur final. Le rapport Si/Al global de ces zéolithes est compris entre 2,6 et 200 de préférence entre 5 et 100 et de manière encore plus préférée entre 5 et 80.

La matrice du catalyseur est un support choisi, par exemple, dans le groupe formé par l'alumine, la silice, la silice-alumine. L'alumihe-oxyde de bore, la magnésie, la silice-magnésie, le zircone, l'oxyde de titane, l'argile, ces composés étant utilisés seuls ou en mélanges. De manière préférée, on utilise un support d'alumine.

La surface B.E.T. du catalyseur n°2 utilisé dans l'étape b) est comprise entre 30 et 900 m²/g de préférence entre 150 et 500 m²/g. Le rapport Na/Al du catalyseur final est inférieur à 5% atomique et de préférence inférieur à 2%.

Comme pour le catalyseur n°1, la zéolithe peut éventuellement être dopée par un promoteur choisi parmi des éléments métalliques comme par exemple, les métaux de la famille des terres rares, notamment le lanthane et le cérium, ou les métaux du groupe VIB de la classification périodique des éléments, comme le molybdène, ou des métaux nobles ou non nobles du groupe VIII, comme le platine, le palladium, le ruthénium, le rhodium, l'iridium, le fer et d'autres métaux comme le manganèse, le zinc, le magnésium.

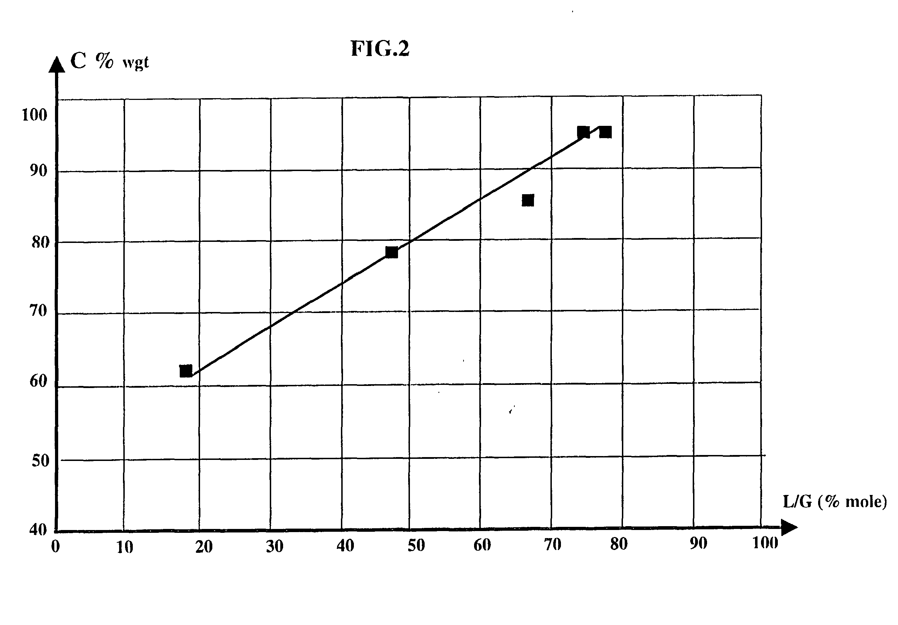

Les étapes a et b, par exemple, se font dans les conditions opératoires suivantes. La pression est comprise entre 2 bars et 90 bars mais de préférence entre 5 et 60 bars. La pression doit être avantageusement la plus haute possible car elle permet à la réaction de se dérouler en phase liquide. La figure 2 montre qu'il y a un lien direct entre la disparition de l'agent alkylant due à l'alkylation de l'aromatique (ou conversion) et le rapport gaz/liquide à l'entrée du réacteur. Dans la figure 2, l'agent alkylant est du décène-1.

Les températures des catalyseurs n°1 et n°2 sont comprises généralement entre 30°C et 300°C et préférentiellement entre 60 et 250°C. La température possède une influence favorable sur la cinétique de la réaction d'alkylation.

La vitesse volumique horaire qui est le rapport du débit de charge sur la volume de catalyseur peut être comprise entre 0.1 m³/m³/h et 100 m³/m³/h et de préférence entre 0,1 et 10 m³/m³/h.

Le rapport aromatique/ agent alkylant est, par exemple, compris entre 0,1 mole/mole et 20 mole/mole et préférentiellement entre 0,1 mole/mole et 10 mole/mole. Le rapport molaire aromatique/agent alkylant possède une influence sur la structure des produits. Le tableau 2 présente pour une conversion de l'oléfine (décène-1) de 100% poids, les rendements en mono et dialkyl benzène varient selon le rapport molaire benzène/décène-1. Le catalyseur utilisé dans ce cas-là est le catalyseur A (tableau 1).

**Tableau 2 :Influence du rapport molaire benzène/oléfines sur les rendements en mono et dialkyl benzène**

| | | | | | | |
|---|---|---|---|---|---|---|
| Rapport molaire | 11,04 | 9,04 | 5,66 | 2,92 | 1 | 0,5 |
| Benzène/Oléfines | | | | | | |
| Bilans matières (% Poids) | | | | | | |
| Benzène (%pds) | 76,9 | 74,4 | 63,3 | 43,1 | 17 | 9 |
| Monoalkyl (%pds) | 22,2 | 24,1 | 33 | 46,2 | 60 | 64 |
| Dialkyl (%pds) | 0,9 | 1,5 | 3,7 | 10,7 | 23 | 27 |
| Conversion oléfines (%pds) | 100 | 100 | 100 | 100 | 100 | 100 |
| Sélectivité en monoalkyl (%) | 96,1 | 94,1 | 89,9 | 81,2 | 72,9 | 70,3 |
| Sélectivité en dialkyl (%) | 3,9 | 5,9 | 10,1 | 18,8 | 27,7 | 29,7 |

Ci-dessous sont présentés quelques exemples du procédé permettant de mieux comprendre son fonctionnement. Dans ces exemples, deux oléfines sont utilisées, il s'agit du décène-1 et de l'octadécène-1. Ces composés peuvent, en fonction des besoins, être remplacés par n'importe quelle oléfine comportant de 2 à 20 atomes de carbone.

### Exemple 1 : Il est souhaité produire conjointement des monoalkyl benzène en C10 et des dialkyl benzène avec une chaîne en C10 et une chaîne en C18.

Le premier réacteur contient 100 m³ de catalyseur D (voir tableau n°1), composé à 70% de zéolithe Nu-87 et 30% d'alumine. Ce réacteur fonctionne à une pression de 40 bars avec un débit de 100 m³ de charge par heure. La charge est composé de 48,86 tonnes de benzène et de 35,08 tonnes de décène-1 ce qui correspond à un rapport molaire benzène sur oléfines de 2,5 moles par mole. Le catalyseur D fonctionne à une température, de 190°C.
Les produits sortant de l'étape a) sont constitués de :
- 18,96 tonnes de décène-1,
- 39,81 tonnes de benzène,
- et de 25,18 tonnes de monoalkyl benzène en C10.

Le benzène et le décèné sont recyclés à l'entrée du réacteur. Des appoints en benzène (9,056 tonnes) et en décène-1 (16,124 tonnes) seront effectués afin de permettre à l'unité de fonctionner en continu.

Pour les monoalkyls, deux choix sont possibles. S'il n'est pas souhaité produire des dialkyls benzène, le deuxième réacteur est arrèté et le produit stocké.

Sinon, les 25,18 tonnes de monoalkyls sont alors envoyés dans l'étape b). Ces alkyls sont mélangés à 23,12 tonnes d'octadécène-1 ce qui correspond à un rapport molaire alkyl/oléfine de 1,26 moles/mole. Le mélange est envoyé dans le second réacteur fonctionnant avec le catalyseur B (voir tableau n°1), constitué à 80% de Mordénite et 20% d'alumine. La pression opératoire est de 60 bars et la température du catalyseur de 210°C. La vitesse volumique horaire est de 1 m³/m³/h.

Les produits sortant de l'étape b) après séparation et distillation sont constitués de :
- 20,6 tonnes d'octadécène-1,
- 22,9 tonnes de monoalkyl benzène en C10,
- 4,8 tonnes de dialkylbenzène en C10-C18.
L'octadécène-1 pourra être recyclé à l'entrée du deuxième réacteur après un appoint de 2,52 tonnes.

Le tableau 3 présente le bilan matière des produits entrant dans l'unité et les produits sortant.

**Tableau 3 : Bilan matière des réactifs consommés et des produits du procédé**

| | Réactifs consommés | Produits |
|---|---|---|
| | (tonnes/h) | (tonnes/h) |
| Benzène | 9,056 | |
| Décène-1 | 16,124 | |
| Octadécène-1 | 2,52 | |
| Monoalkyl benzène (C10) | | 22,9 |
| Diakyl benzène (C10-C18) | | 4,8 |
| Total | 27,7 | 27,7 |

### Exemple 2 : Il est souhaité produire conjointement des monoalkyl benzène en C10, et deux types différents de dialkyls :

- des dialkyl benzène avec deux chaînes en C10,
- des dialkyls benzène avec une chaîne en C10 et une chaîne en C18.

Le premier réacteur contient du catalyseur B, composé à 80% de Mordénite et 20% d'alumine. Ce réacteur fonctionne à une pression de 60 bars avec un débit permettant d'obtenir une vitesse volumique horaire de 1 m³/m³/h. La charge est composé de 48,86 tonnes de benzène et de 35,08 tonnes de décène-1 ce qui correspond à un rapport molaire benzène sur oléfines de 2,5 moles par mole. Le catalyseur D fonctionne à une température de 180°C.

Les produits sortant de l'étape a) sont constitués de :
- 0,09 tonnes de décène-1,
- 35,43 tonnes de benzène,
- 20,31 tonnes de monoalkyl benzène en C10,
- 28,12 tonnes de dialkyl en C10 (C10-C10).

Le benzène et le décène sont recyclés à l'entrée du réacteur. Des appoints en benzène (13,436 tonnes) et en décène-1 (34,994 tonnes) seront effectués afin de permettre à l'unité de fonctionner en continu.

Si il est souhaité produire uniquement des monoalkyls et des dialkyls, le deuxième réacteur est arrèté et les produits stockés. Si uniquement des monoalkyls sont souhaités, les dialkyls en C10-C10 sont recyclés à l'entrée du premier réacteur pour subir une transalkylation avec le benzène. Si ce sont uniquement des dialkyls benzène symétriques qui sont désirés, les monalkyls sont recyclés à l'entrée du premier réacteur pour subir une seconde alkylation.

Sinon, les 28,12 tonnes de dialkyls en C10-C10 sont stockés. Les 20,31 tonnes de monoalkyls sont alors envoyés dans l'étape b). Ces alkyls sont mélangés à 18,64 tonnes d'octadécène-1 ce qui correspond à un rapport molaire alkyl/oléfine de 1,26 moles/mole. Le mélange est envoyé dans le second réacteur fonctionnant avec le catalyseur B, constitué à 80% de Mordénite et 20% d'alumine. La pression opératoire est de 60 bars et la température du catalyseur de 210°C. La vitesse volumique horaire est de 1 m³/m³/h.

Les produits sortant de l'étape b) après séparation et distillation sont constitués de :
- 16,55 tonnes d'octadécène-1,
- 18,50 tonnes de monoalkyl benzène en C10,
- 3,9 tonnes de dialkylbenzène en C10-C18.

L'octadécène-1 pourra être recyclé à l'entrée du deuxième réacteur après un appoint de 2,09 tonnes.

Le tableau 4 présente le bilan matière des produits entrant dans l'unité et les produits sortant.

**Tableau 4 : Bilan matière du procédé dans l'exemple 2.**

| | Réactifs consommés | Produits |
|---|---|---|
| | (tonnes/h) | (tonnes/h) |
| Benzène | 13,436 | |
| Décène-1 | 34,994 | |
| Octadécène-1 | 2,09 | |
| Monoalkyl benzène (C10) | | 18,50 |
| Diakyl benzène (C10-C10) | | 28,12 |
| Diakyl benzène (C10-C18) | | 50,52 |
| Total | 50,52 | 27,7 |

## Revendications

1. Procédé pour la fabrication d'au moins un composé choisi parmi les composés monoalkylaromatiques, dialkylaromatiques et trialkylaromatiques par alkylation ou transalkylation d'un composé aromatique par au moins un agent alkylant choisi parmi les oléfines, le procédé étant **caractérisé en ce qu'**il est effectué en deux étapes en série, chaque étape mettant en oeuvre une zone de réaction dans laquelle les aromatiques réagissent avec un agent alkylant, et **en ce que** l'une de ces deux étapes peut être mise en oeuvre ou mise hors circuit pour pouvoir fournir à la demande des monoalkylaromatiques, des dialkylaromatiques pour lesquels les chaînes aliphatiques sont de longueurs différentes ou identiques ou des trialkylaromatiques pour lesquels les chaînes aliphatiques sont de même longueur ou pour lesquels deux chaînes aliphatiques sont de longueurs différentes.

2. Procédé selon la revendication 1 dans lequel la chaîne aliphatique des composés monoalkylaromatiques, dialkylaromatiques et trialkylaromatiques fabriqués comporte de 2 à 20 atomes de carbone.

3. Procédé selon la revendication 1 ou 2 dans lequel les composés monoalkylaromatiques issus de la première étape alimentent la zone de réaction de la deuxième étape.

4. Procédé selon l'une des revendications 1 à 3 dans lequel les zones de réaction contiennent des catalyseurs différents.

5. Procédé selon l'une des revendications 1 à 4 dans lequel les zones de réaction sont alimentées par des oléfines dont les longueurs de chaîne sont différentes.

6. Procédé selon l'une des revendications 1 à 5 où les pressions opératoires sont comprises entre 2 et 90 bars, préférentiellement comprises entre 5 et 60 bars ; où les températures opératoires sont comprises entre 30 et 300 °C, préférentiellement comprises entre 60 et 250 °C.

7. Procédé selon l'une des revendications 1 à 6 où les vitesses volumiques horaires sont comprises entre 0,1 et 100 m³/m³/h, préférentiellement comprises entre 0,1 et 10 m³/m³/h.

8. Procédé selon l'une des revendications 1 à 7 où, dans chaque zone de réaction, les rapports molaires aromatiques sur agent alkylant sont compris entre 0,1 et 20 moles et préférentiellement compris entre 0,1 et 10 moles/mole.

9. Procédé selon l'une des revendications 1 à 8 où les catalyseurs sont constitués de solides acides.

10. Procédé selon la revendication 9 où les catalyseurs contiennent entre 5 et 95% poids de zéolithe.

11. Procédé selon la revendication 10 où la zéolithe est de type faujasite (zéolithe Y), mordénite, EUO, EU-1, ZSM-50, TPZ-3, NU-87, NU-88, NU-86, IM-5.

12. Procédé selon la revendication 11 où la zéolithe peut être dopée par au moins un promoteur choisi dans le groupe constitué par des métaux de la famille des terres rares notamment le lanthane et le cérium, par des métaux du groupe VIB de la classification des éléments comme le molybdène, par des métaux nobles ou non nobles du groupe VIII comme le platine , le palladium, le ruthénium, le rhodium, l'iridium, le fer et par d'autres métaux comme le manganèse, le zinc, le magnésium.

13. Procédé selon la revendication 1 **caractérisé en ce qu'**il comprend :
- une première mise en contact dans une première zone d'alkylation d'un composé aromatique avec un premier agent alkylant choisi parmi les oléfines, suivi d'une première séparation des effluents de la réaction en au moins trois fractions dont :
des monoalkylaromatiques,
des dialkylaromatiques symétriques, c'est à dire comprenant des chaînes aliphatiques de longueur identique,
les réactifs n'ayant pas réagi,
au moins une fraction desdits monoalkylaromatiques ou desdits dialkylaromatiques constituant un produit de la réaction,
- une deuxième mise en contact dans une seconde zone d'alkylation d'au moins une fraction des monoalkylaromatiques issus de la première zone d'alkylation avec un deuxième agent alkylant choisi parmi les oléfines, suivi d'une seconde séparation des effluents de la réaction en au moins trois fractions dont :
- les monoalkylaromatiques n'ayant pas réagi,
- des dialkylaromatiques asymétriques, c'est à dire comprenant des chaînes aliphatiques de longueurs différentes,
- des trialkylaromatiques asymétriques, c'est à dire comprenant des chaînes aliphatiques de longueur différentes,
au moins une fraction desdits monoalkylaromatiques, desdits dialkylaromatiques ou desdits trialkylaromatiques constituant un produit de la réaction.

14. Procédé selon la revendication 13 dans lequel au moins une partie des monoalkylaromatiques issus de la première mise en contact est recyclée dans ladite première zone.

15. Procédé selon la revendication 13 ou 14 dans lequel au moins une partie des dialkylaromatiques issus de la première mise en contact est recyclée dans ladite première zone.

16. Procédé selon l'une des revendications 13 à 15 dans lequel au moins une partie des monoalkylaromatiques issus de la deuxième mise en contact est recyclée dans ladite deuxième zone.

17. Procédé selon l'une des revendications 13 à 16 dans lequel au moins une partie des dialkylaromatiques issus de la deuxième mise en contact est recyclée dans ladite deuxième zone.

18. Procédé selon l'une des revendications 13 à 17 dans lequel au moins une partie desdits trialkylaromatiques issus de la deuxième mise en contact est recyclée dans ladite deuxième zone.

## Claims

1. Process for the production of at least one compound that is selected from among monoalkyl aromatic compounds, dialkyl aromatic compounds and trialkyl aromatic compounds by alkylation or transalkylation of an aromatic compound by at least one alkylating agent that is selected among olefins, whereby the process is **characterized in that** it is carried out in two stages in series, each step involving one reaction zone in which aromatics react with an alkylating agent, and **in that** one of these two steps can be restarted or switched off so as to be able to meet the demand of monoalkyaromatics, dialkylaromatics for which the aliphatic chains are of different or identical lengths or trialkylaromatics for which the aliphatic chains are of the same length or for which two aliphatic chains are of different lengths.

2. Process according to claim 1, wherein the aliphatic chain of produced monoalkyaromatics, dialkylaromatics and trialkylaromatics comprises 2 to 20 carbon atoms.

3. Process according to one of claims 1 and 2 wherein monoalkyaromatics from the first stage supplies the reaction zone of the second stage.

4. Process according to one of claims 1 to 3 wherein the reactions zones comprise different catalysts.

5. Process according to one of claims 1 to 4 wherein the reaction zones are supplied by olefins having different chain lengths.

6. Process according to one of claims 1 to 5 wherein the operating pressures are between 2 and 90 bars, preferably between 5 and 60 bars; wherein the operating temperatures are between 30 and 300°C, preferably between 60 and 250°C.

7. Process according to claims 1 to 6, wherein the hourly volumetric flow rates are between 0,1 and 100 m³/m³/h, preferably between 0,1 and 10 m³/m³/h.

8. Process according to claims 1 to 7, where, in each reaction zone, the aromatic compounds to alkylating agent molar ratios are between 0,1 and 20 mol and preferably between 0,1 and 10 mol/mol.

9. Process according to claims 1 to 8, where the catalysts consist of acidic solids.

10. Process according to claim 9, where the catalysts contain between 5 and 95% by weight of zeolite.

11. Process according to claim 10, where the zeolite is of type faujasite (Y zeolite), mordenite, EUO, EU-1, ZSM-50, TPZ-3, NU-87, NU-88, NU-86, or IM-5.

12. Process according to claim 11, where the zeolite can be doped by at least one promoter that is selected from the group that consists of metals of the family of rare earths, in particular lanthanum and cerium, by metals of group VIB of the periodic table, such as molybdenum, by noble metals or non-noble metals of group VIII, such as platinum, palladium, ruthenium, rhodium, iridium, iron, and by other metals such as manganese, zinc or magnesium.

13. Process according to claim 1 comprising :
- a first contacting in a first alkylation zone of an aromatic compound with a first alkylating agent selected among olefins followed by a first separation of the reaction effluents in at least three fractions :
- monoalkylaromatics,
- symmetrical dialkylaromatics, i.e. comprising aliphatic chains of identical lengths,
- non-reacted reagent,
at least one fraction of said monoalkylaromatics or said dialkylaromatics forming a reaction product,
- a second contacting in a second alkylation zone of at least one monoalkylaromatics fraction from the first alkylation zone with a second alkylating agent selected among olefins followed by a second separation of the reaction effluents in at least three fractions :
- non-reacted monoalkylaromatics,
- non-symmetrical dialkylaromatics, i.e. comprising aliphatic chains of different lengths,
- non-symmetrical trialkylaromatics, i.e. comprising aliphatic chains of different lengths,
at least one fraction of said monoalkylaromatics, said dialkylaromatics or said trialkylaromatics forming a reaction product.

14. Process according to claim 13 in which at least a part of monoalkylaromatics from the first contacting is recycled to said first zone.

15. Process according to claim 13 or claim 14 in which a part of dialkylaromatics from the first contacting is recycled to said first zone.

16. Process according to one of claims 13 to 15 in which a part of monoalkylaromatics from the second contacting is recycled to said second zone.

17. Process according to one of claims 13 to 16 in which a part of dialkylaromatics from the second contacting is recycled to said second zone.

18. Process according to one of claims 13 to 17 in which at least a part of said trialkylaromatics from the second contacting is recycled to said second zone.

## Patentansprüche

1. Verfahren zur Herstellung wenigstens einer Verbindung, gewählt unter den monoalkylaromatischen, dialkylaromatischen und trialkylaromatischen Verbindungen, durch Alkylierung oder Transalkylierung einer aromatischen Verbindung durch wenigstens ein alkylierendes Mittel, gewählt unter den Olefinen, wobei das Verfahren **dadurch gekennzeichnet ist, dass** es in zwei Stufen in Reihe durchgeführt wird, wobei jede Stufe eine Reaktionszone einsetzt, in welcher die Aromaten mit einem Alkylierungsmittel reagieren und **dadurch**, dass die einer dieser beiden Stufen eingesetzt oder ausgeschaltet werden können, um die Nachfrage der Monoalkylaromaten, der Dialkylaromaten zu beliefern, für welche die Aliphatenketten von unterschiedlichen oder identischen Längen sind, oder der Trialkylaromaten, für die die aliphatischen Kettenlängen von gleicher Länge sind oder für die die aliphatischen Ketten von unterschiedlichen Längen sind.

2. Verfahren nach Anspruch 1, bei dem die aliphatische Kette der Monoalkylaromaten-, Dialkylaromatenverbindungen, die hergestellt sind, 2 bis 20 Kohlenstoffatome aufweist.

3. Verfahren nach Anspruch 1 oder 2, bei dem die Monoalkylaromatenverbindungen aus der ersten Stufe die Reaktionszone der zweiten Stufe speisen.

4. Verfahren nach einem der Ansprüche 1 bis 3, bei dem die Reaktionszonen verschiedene Katalysatoren enthalten.

5. Verfahren nach einem der Ansprüche 1 bis 4, bei dem die Reaktionszonen durch Olefine gespeist werden, deren Kettenlängen unterschiedlich sind.

6. Verfahren nach einem der Ansprüche 1 bis 5, wo die Betriebsdrucke zwischen 2 und 90 bar, vorzugsweise zwischen 5 und 60 bar liegen, wobei die Betriebstemperaturen zwischen 30 und 300°C, vorzugsweise zwischen 60 und 250°C liegen.

7. Verfahren nach einem der Ansprüche 1 bis 6, wobei die stündlichen Volumengeschwindigkeiten zwischen 0,1 und 100 m³/m³/h, vorzugsweise zwischen 0,1 und 10 m³/m³/h liegen.

8. Verfahren nach einem der Ansprüche 1 bis 7, wobei in jeder Reaktionszone die molaren Verhältnisse von Aromaten zu Alkylierungsmittel zwischen 0,1 und 20 mol und vorzugsweise zwischen 0,1 und 10 mol/mol liegen.

9. Verfahren nach einem der Ansprüche 1 bis 8, wobei die Katalysatoren aus sauren Feststoffen bestehen.

10. Verfahren nach Anspruch 9, wobei die Katalysatoren zwischen 5 und 95 Gew.-% Zeolith enthalten.

11. Verfahren nach Anspruch 10, wobei der Zeolith vom Typ Faujasit (Y-Zeolith), Mordenit, EUO, EU-1, ZSM-50, TPZ-3, NU-87, NU-88, NU-86, IM-5 ist.

12. Verfahren nach Anspruch 11, wobei der Zeolith durch wenigstens einen Promotor dotiert sein kann, der gewählt ist aus der Gruppe, die besteht aus den Metallen der Familie der Seltenerden, insbesondere Lanthan und Cer, durch die Metalle der Gruppe VIB des Periodensystems der Elemente wie Molybdän, durch die edlen oder nicht-edlen Metalle der Gruppe VIII wie Platin, Palladium, Ruthenium, Rhodium, Iridium, Eisen und durch andere Metalle wie Mangan, Zink und Magnesium.

13. Verfahren nach Anspruch 1, **dadurch gekennzeichnet, dass** es umfasst:
- ein erstes Kontaktieren in einer Alkylierungszone einer aromatischen Verbindung mit einem ersten Alkylierungsmittel, gewählt unter den Olefinen, gefolgt von einer ersten Trennung der Abströme der Reaktion in wenigstens drei Fraktionen, darunter:
Monoalkylaromaten,
Dialkylaromaten, die symmetrisch sind, das heißt, welche aliphatische Ketten identischer Länge umfassen;
Reagenzien, die nicht reagiert haben,
wenigstens eine Fraktion der Monoalkylaromaten oder der Dialkylaromaten bildet dabei das Reaktionsprodukt,
ein zweites Kontaktieren in einer zweiten Alkylierungszone wenigstens einer Fraktion der Monoalkylaromaten aus der ersten Alkylierungszone mit einem zweiten Alkylierungsmittel, gewählt unter den Olefinen, gefolgt von einer zweiten Trennung der Abströme der Reaktion in wenigstens drei Fraktionen, darunter:
- die Monoalkylaromaten, die nicht reagiert haben,
die Alkylaromaten, die asymmetrisch sind, das heißt, welche aliphatische Ketten verschiedener Längen umfassen,
Trialkylaromaten, die asymmetrisch sind, das heißt, welche aliphatische Ketten umfassen mit verschiedenen Längen,
wenigstens eine Fraktion der Monoalkylaromaten, der Dialkylaromaten oder der Trialkylaromaten, die das Reaktionsprodukt bildet.

14. Verfahren nach Anspruch 13, bei dem wenigstens ein Teil der Monoalkylaromaten aus dem ersten Kontaktieren in die erste Zone rezykliert wird.

15. Verfahren nach Anspruch 13 oder 14, bei dem wenigstens ein Teil der Dialkylaromaten aus dem ersten Kontaktieren in die erste Zone rezykliert wird.

16. Verfahren nach einem der Ansprüche 13 bis 15, bei dem wenigstens ein Teil der Monoalkylaromaten aus dem zweiten Kontaktieren in die zweite Zone rezykliert wird.

17. Verfahren nach einem der Ansprüche 13 bis 16, bei dem wenigstens ein Teil der Dialkylaromaten aus dem zweiten Kontaktieren in die zweite Zone rezykliert wird.

18. Verfahren nach einem der Ansprüche 13 bis 17, bei dem wenigstens ein Teil der Trialkylaromaten aus dem zweiten Kontaktieren in die zweite Zone rezykliert wird.
